Europäisches Patentamt

European Patent Office

Office européen des brevets

(11) Publication number: **0 357 776**
**A1**

## EUROPEAN PATENT APPLICATION
### published in accordance with Art. 158(3) EPC

(21) Application number: 88904267.7

(22) Date of filing: 13.05.88

(86) International application number:
PCT/JP88/00460

(87) International publication number:
WO 88/08853 (17.11.88 88/25)

(51) Int. Cl.5 **C07K 15/00 , C12P 21/06 ,
A23J 3/00 , //A23L1/06,
A23L1/24,A23L1/187,A23L2/38,
A21D13/08,A23G3/00**

(30) Priority: 14.05.87 JP 115758/87
14.05.87 JP 115759/87
14.05.87 JP 115760/87

(43) Date of publication of application:
**14.03.90 Bulletin 90/11**

(84) Designated Contracting States:
**BE DE FR GB IT NL**

(71) Applicant: **Terumo Kabushiki Kaisha
No. 44-1, Hatagaya 2-chome Shibuya-ku
Tokyo 151(JP)**

(72) Inventor: **SUZUKI, Seiji Terumo Kabushiki
Kaisha
1727-1, Tsukijiarai Showa-cho
Nakakoma-gun Yamanashi 409-38(JP)**
Inventor: **SHIOTA, Junichiro Terumo
Kabushiki Kaisha
1727-1, Tsukijiarai Showa-cho
Nakakoma-gun Yamanashi 409-38(JP)**

(74) Representative: **Gillard, Marie-Louise et al
Cabinet Beau de Loménie 55, Rue
d'Amsterdam
F-75008 Paris(FR)**

(54) **EGG WHITE HYDROLYZATE.**

(57) A lowly antigenic hydrolyzate of protein, particularly that of egg white, is disclosed. This hydrolyzate is substantially free from ovoalbumin and ovomucoid, and contains at least 95 wt % of a protein hydrolyzate having a molecular weight distribution of 2,800 to 18,000 as determined by gel chromatography. It has an antigenicity half that of egg white per unit protein as measured according to the ELISA method using anti-egg white serum. This hydrolyzate can be obtained by heating a proteinous substance in a polar hydrophilic organic solvent to denature the protein and hydrolyzing the product with protease of bacterial origin.

S P E C I F I C A T I O N

Albumen Hydrolyzates

Technical Field

The present invention relates to albumen hydrolyzates. In particular, it is concerned with antigenicity-reduced albumen hydrolyzates which are suitable for use as protein source in food-allergic patients of protein origin, sucklings and little children as well as pregnant women and nursing mothers for prophylactic purpose.

Furthermore, the invention relates to a method for preparing antigenicity-reduced protein hydrolyzates.

Background Art

Most of the food allergy-causing antigens are proteins. Food allergic patients when given such antigen-containing food develop allergic symptoms such as asthma, atopic dermatitis and diarrhea. Higher incidences of the food allergy in sucklings and little children are supposed to be due to various caused including premature digestive tract mucosas, immunological prematurity as well as ingestion of a single food for a long term. It is further known that if a pregnant woman at a later stage of pregnancy receives such antigen, the antigen is transferred to the fetus via blood or migrated to the suckling via milk so that the fetus or the suckling is indirectly sensitized to food allergy. (Ogura et al. "Arerugi" (Allergy) 35, 842 (1986)).

Therefore, receiving antigen-containing meal that will cause food allergy will be dangerous not only in patients who are allergic to the food but also in sucklings, little children and women before and after delivery.

Among the proteins, albumen protein frequently causes food allergy. However, there have been very few attempts to reduce antigenicity of the same. Only Japanese Patent Application Open-to-Public No. 15644/1986 discloses reduction of the antigenicity of albumen by means of heat denaturation with an acid.

The antigenicity of albumin is attributed to ovalbumin, ovomucoid and lysozyme in the albumen protein. Among them antigenic activity of ovomucoid is believed to be the highest. The ovomucoid, however, is hardly decomposable in the digestive tract and, as reported by Matsuda et al. "Biochemica et Biophysica Acta" 707, 121-128 (1982), cannot undergo marked reduction of the antigenicity even by heat treatment. This may reflect the fact that the antigenic determinant of ovomucoid is largely dependent upon amino acid sequence or primary structure which is not affected by conformational changes by physicochemical treatment such as heat, acid or alkali treatment. Accordingly, it is conceivable that marked reduction in antigenicity would not be achieved by the physicochemical treatment alone. As a matter of fact, results of our test show that denatured albumen produced either by the heating method with an acid as disclosed in the above-mentioned application or by a treatment with ethanol at 75°C possesses a high antigenicity (see Table 8).

Thus, it can be said that marked reduction in antigenicity of albumen would be unachievable without hydrolysis of the albumen.

Such being the case, it may follow that enzymatic hydrolysis is effective for reduction of the antigenicity of albumen. Nevertheless, no success is presently known in efficiently achieving production of antigen-reduced albumen.

Whereas a number of enzymatic hydrolyses of protein are known, most of them are directed to production of low molecular weight proteins free of unpleasant taste as nutritional supplement. For example, there are proposed a method for producing soluble proteins by enzymatic hydrolysis of albumen followed by heat denaturation of macromolecular substances to remove them (Japanese Patent Application Laid-Open-to-Public No. 38579/1982, No. 124458/1980 and others); a method for enzymatic hydrolysis of albumen after heat denaturation (Japanese Patent Application Laid-Open-to-Public No. 158138/1983, No. 158137/1983 and others); and a method of the enzymatic hydrolysis using two or more proteases. We have investigated these methods and found that none of them can be suitable for efficiently producing proteins or peptides

EP 0 357 776 A1

with antigenicity eliminated.

Disclosure of the Invention

As a result of extensive studies we have found that combination of heat denaturation with a polar hydrophilic organic solvent and enzymatic hydrolysis enables removal of antigenic determinants in proteins. The present invention has been completed on the basis of the above finding.

It is an object of the invention to provide albumen hydrolyzates having very low antigenicity.

A further object of the invention is to provide a method for preparing antigen-reduced protein hydrolyzate.

According to the invention there are provided albumen hydrolyzates and a method for preparing protein hydrolyzates as set forth below.

1) An albumen hydrolyzate substantially free of ovalbumin and ovomucoid and containing 95% by weight or more of a protein hydrolyzate of a molecular weight distribution in a range of 2,800 to 18,000 as determined by gel chromatography.

2) An albumen hydrolyzate with antigenicity reduced to a half or below of that of the albumen per unit protein as determined by ELISA (enzyme linked immunosorbent assay) method using anti-albumen serum.

3) An antigenicity-reduced food comprising an albumen hydrolyzate according to item 1 or 2.

4) A method for preparing protein hydrolyzates which comprises denaturing a proteinous substance by heating in a polar hydrophilic organic solvent followed by hydrolysis with a bacterial protease.

5) A method for preparing protein hydrolyzates according to item 4 wherein the heating temperature is 50-95°C.

6) A method for preparing protein hydrolyzates according to item 4 wherein the proteinous substance is albumen protein.

7) A method for preparing protein hydrolyzates according to item 4 wherein the proteinous substance is dairy protein.

8) A method for preparing protein hydrolyzates according to item 4 wherein the proteinous substance is cereal protein.

9) A method for preparing protein hydrolyzates according to item 4 wherein the proteinous substance is meat protein.

10) A preparative method according to item 4 wherein the polar hydrophilic organic solvent is a lower alcohol.

11) A preparative method according to item 4 wherein the bacterial protease is a heat-resistant neutral protease.

12) A method for preparing protein hydrolyzates which comprising denaturing a proteinous substance by heating in a polar hydrophilic solvent followed by hydrolysis with a bacterial protease, subjecting the hydrolyzate to centrifugation and collecting the supernatant.

13)     A method for preparing protein hydrolyzates according to item 12 wherein the heating temperature is 50-95°C.

In the present invention protein hydrolyzates are prepared by denaturing a proteinous substance by heating in a polar hydrophilic organic solvent at 50-95°C followed by hydrolysis with a bacterial protease, subjecting the hydrolyzate to centrifugation and isolating the hydrolyzate from the supernatant.

The proteinous substance used in the invention is plant proteins, for example, beans such as soybean and cereals such as wheat (wheat gluten) and rice and animal proteins, for example, albumen, dairy products such as powdery skim milk, cow milk and whey and meats such as fish meat.

Animal proteins, especially albumen protein is preferably used.

The polar hydrophilic organic solvent is desirably a lower alcohol. For example, methanol, ethanol, 1-propanol, 2-propanol, 1-butanol, 2-butanol, 2-methyl-2-propanol, 1-pentanol and 3-methyl-1-butanol are mentioned. Ethanol is particularly preferable.

The bacterial protease used in the invention is desirably neutral proteases such as Panchidase NP-2 (manufactured by Yakult K.K.), Protease Amano "A" and "P" (manufactured by Amano Seiyaku K.K.) and Protein P (manufactured by Yamato Kasei K.K.). Especially desirable are heat-resistant neutral proteases such as thermoase (manufactured by Yamato Kasei K.K.).

(Preparative Examples)

Preparative examples of the protein hydrolyzates according to the invention will concretely be described below.

To a lower alcohol, especially ethanol or an aqueous ethanol, preferably 5-80% aqueous ethanol is added albumen to a protein concentration of, for example, 1-15%, preferably 3-8% while stirring, the concentration depending on nature of the starting protein. The mixture is then heated with stirring at 50-95°C, preferably 65-85°C for 5-120 min., preferably 20-60 min. The heat-denaturation step with organic solvent is very effective for efficiently conducting subsequent enzymatic hydrolysis step and also for obtaining an antigenicity-free hydrolyzate.

Next, the ethanol is removed by warming under a reduced pressure, and the residue is adjusted with water to a protein concentration of 1-10%, preferably 2-8%. To the denatured albumen suspension is added a neutral protease to conduct hydrolysis. The amount to be added is, for example, in the case of thermoase preferably 0.01-1% on the basis of substrate. The reaction time is variable depending upon the starting protein, and in general it is 15 min.-10 hours and particularly preferably 30 min.-4 hours for albumen, 1 hour or longer for powdery skim milk, or 4 hours or longer for whey. Longer reaction time will cause unfavorable decomposition to amino acids with a result that the hydrolyzate obtained tastes bitter.

The albumen hydrolyzate thus produced is subjected to centrifugation, and the supernatant is dried using a spray drier, a freeze drier or the like to obtain a powdery protein hydrolyzate.

In addition, separation means other than the centrifugation such as filtration may also be employed.

It is noted that content of free amino acids in the hydrolyzates of the invention is 1.5% or below.

Higher levels of free amino acids will produce bitter taste in the protein, but the albumen hydrolyzates of the invention are almost free of bitter taste as the free amino-acid level is so low.

Example 1

Through a 500μ-mesh screen was passed 150 g of a commercially available dry albumen (manufactured by Taiyo Kagaku K.K.) which was then suspended with vigorous stirring in 3 lit. of 10% aqueous ethanol. The suspension was placed in a separable flask equipped with a cooler and was heated at 75°C for 1 hour with stirring at 400 rpm. After completion of the heating the ethanol was removed in a rotary evaporator followed by dilution with water to a fixed volume of 3 lit. (at a pH of about 7). The mixture was heated to 70°C to which was then added 3 g of Thermoase PC10F (Yamato Kasei) followed by hydrolysis with stirring. The resultant albumen hydrolyzate was subjected to a centrifugation at 3,000 rpm for 10 min. to separate supernatant. The supernatant was freeze-dried to give solids. As shown in Table 1, protein recovery ratio for the solids is increased as hydrolysis time is longer. However, as the product hydrolyzed for 6 hours or longer tastes somewhat bitter, the hydrolysis time of about 4 hours is preferable. As seen from the same table, reduction in antigenicity was almost achieved in the hydrolyses conducted for 30 min. or longer.

Table 1

|  | Hydrolysis time | | | | | | |
|---|---|---|---|---|---|---|---|
|  | 0 | 15 min. | 30 min. | 1 hr. | 2 hr. | 4 hr. | 6 hr. |
| Protein recovery ratio (%) | 20.7 | 33.7 | 39.0 | 46.3 | 54.7 | 61.3 | 65.1 |
| Taste | Taste-less | Taste-less | Taste-less | Taste-less | Taste-less | Taste-less | Slightly bitter |
| Antigenicity | 48.0 | 17.2 | 10.0 | 12.8 | 9.0 | 10.8 | 8.5 |

Example 2

In 100 ml of 10% aqueous ethanol was suspended 5 g of a commercially available powder skim milk (manufactured by Kyushu Nyugyo K.K.) with vigorous stirring. The suspension was heated with stirring at 75°C for 1 hour. After completion of the heating the mixture was subjected to centrifugation, and precipitates were re-suspended in 100 ml of distilled water, and the suspension was warmed to 70°C and admixed with 40 mg of Termoase C100 (manufactured by Yamato Kasei K.K.). Hydrolysis of the mixture was effected with stirring. The hydrolyzate thus obtained was centrifuged at 3,000 rpm for 10 min. The supernatant was separated and freeze-dried to give a solid.

As shown in Table 2, the protein recovery ratio was increased as the hydrolysis time was longer. Even with the treatment conducted for 6 hours there was no bitter taste developed. As also seen from the same table, reduction in antigenicity was almost achieved by the treatments conducted for 1 hour or longer.

Table 2

|  | Hydrolysis time | | | | | |
|---|---|---|---|---|---|---|
|  | 0 | 30 min. | 1 hr. | 2 hr. | 4 hr. | 6 hr. |
| Protein recovery ratio (%) | 96 | 99 | 97 | 98 | 98 | 99 |
| Taste | Taste-less | Taste-less | Taste-less | Taste-less | Taste-less | Taste-less |
| Antigeni-city | 82 | 95 | 52 | 48 | 49 | 45 |

Example 3

A commercially available whey (manufactured by Yotsuba Nyugyo K.K.) was hydrolyzed in accordance with the procedures described in Example 2 to obtain hydrolyzates.

As shown in Table 3, protein recovery ratio for the hydrolyzates was increased as the hydrolysis time was longer. Even with the 8-hour treatment there was no bitter taste developed. As also seen from the same table, reduction in antigenicity remarkably progressed in the hydrolyses conducted for 4 hours or longer and was almost achieved in the hydrolysis conducted for 8 hours.

Table 3

| | | | | Hydrolysis time | | | |
|---|---|---|---|---|---|---|---|
| | 0 | 30 min. | 1 hr. | 2 hr. | 4 hr. | 6 hr. | 8 hr. |
| Protein recovery ratio (%) | 24 | 89 | 88 | 87 | 88 | 88 | 89 |
| Taste | Taste-less | Taste-less | Taste-less | Taste-less | Taste-less | Taste-less | Taste-less |
| Antigeni-city | 54 | 59 | 46 | 49 | 27 | 20 | 6 |

Example 4

300 g of pieces of sardine meat with head, tail, bones and viscera together removed together with 2 lit. of distilled water was homogenated in a juicer for 5 min. and was kept standing at 4°C for 2 hours. After centrifugation at 3,000 rpm for 10 min., the solution phase was freeze-dried to afford a dried product of sardine aqueous extract. The product was hydrolyzed in accordance with the procedures described in Example 2 to obtain hydrolyzates.

As shown in Table 4, protein recovery ratio for the hydrolyzates was increased as the hydrolysis time was longer. As it reached nearly plateau in more than one-hour, and no further increase in the recovery ratio was observed in the hydrolyses conducted for a period of time longer than 4 hours, the time is preferably 2-4 hours. As seen from the same table, reduction in antigenicity was almost achieved in the hydrolyses conducted for 1 hour or longer.

Table 4

(Sardine)

|  | Hydrolysis time | | | | | |
|---|---|---|---|---|---|---|
|  | 0 | 30 min. | 1 hr. | 2 hr. | 4 hr. | 6 hr. |
| Protein recovery ratio (%) | 19 | 38 | 42 | 45 | 46 | 46 |
| Taste | Taste-less | Taste-less | Taste-less | Taste-less | Taste-less | Taste-less |
| Antigeni-city | 18 | 18 | 2 | 2 | 2 | 2 |

Example 5

A suspension of 300 g of powdery soybean which had been pulverized by an electric mill to a size of $\phi$250 µm or shorter in 2 lit. of distilled water was allowed to stand at 4°C for 2 hours. The liquid was then centrifuged at 3,000 rpm for 10 min., and the solution phase was freeze-dried to give a dried product of soybean aqueous extract. The product was hydrolyzed in accordance with the procedures described in Example 2 to obtain hydrolyzates.

As shown in Table 5, protein recovery ratio for the hydrolyzates is increased as the hydrolysis time is longer. However, as the products hydrolyzed for 2 hours or longer taste somewhat bitter, hydrolysis conducted for about 1 hour is preferable. As also seen from the same table, reduction in antigenicity was almost achieved by the hydrolyses conducted for 30 min. or longer.

Table 5

|  | Hydrolysis time | | | | (Soybean) | |
|  | 0 | 30 min. | 1 hr. | 2 hr. | 4 hr. | 6 hr. |
|---|---|---|---|---|---|---|
| Protein recovery ratio (%) | 38 | 71 | 75 | 78 | 82 | 82 |
| Taste | Taste-less | Taste-less | Taste-less | Slightly bitter | Bitter | Bitter |
| Antigeni-city | 14 | 7 | 5 | 2 | 1 | 1 |

Example 6

A dried product from aqueous extract of rice produced in the same way as in Example 5 was hydrolyzed in accordance with the procedures described in Example 5 to obtain hydrolyzates.

Protein recovery ratios in Table 6 indicate that the hydrolysis when conducted for 30 min. or longer is sufficient. As also seen from the same table, reduction in antigenicity is satisfactory by the hydrolyses conducted for 1 hour or longer and is almost achieved by the hydrolyses conducted for 4 hours or longer.

### Table 6

(Rice)

| | Hydrolysis time | | | | | |
|---|---|---|---|---|---|---|
| | 0 | 30 min. | 1 hr. | 2 hr. | 4 hr. | 6 hr. |
| Protein recovery ratio (%) | 48 | 97 | 94 | 94 | 95 | 96 |
| Taste | Slightly sweet | Slightly sweet | Slightly sweet | Slightly sweet | Slightly sweet | Slightly sweet |
| Antigeni-city | 72 | 85 | 10 | 3 | 1 | 1 |

Test Example 1

Results of the tests for molecular weight distribution, amino acid composition and antigenicity of the albumen hydrolyzates of the present invention are shown below.

(1) Molecular weight distribution

Molecular weight distribution of the albumen hydrolyzates of the invention was measured by gel chromatography. Measurement conditions were set as follows:

Sample: Albumen hydrolyzate 10 mg/ml ⟶ 30 μl

Buffer solution: PBS

Column: TSK gel G3000 SWxL

Flow rate: 0.5 ml/min.

Detection: RInm 1/32 range

Record: 30 cm/hr. 1 V 1 ml/cm

As the molecular weight marker were employed conalbumin 77K, ovalbumin 45K, lysozyme 14K, cyanocobalamin 1355 and Ala-Ala-Ala 267.

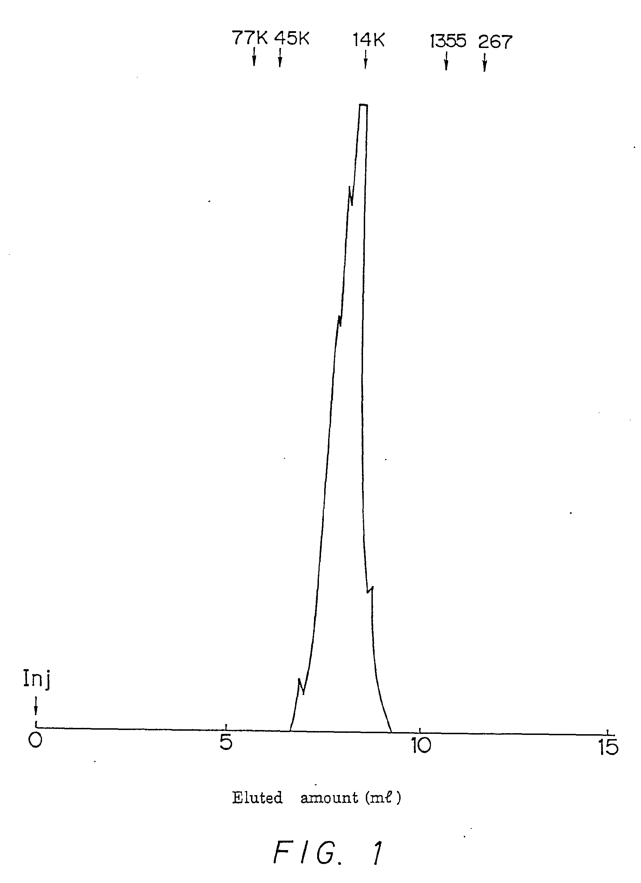

The results are shown in Fig. 1.

As shown in Fig. 1, most of the hydrolyzates, 95% by weight or more of the whole hydrolyzates, have a molecular weight ranging from 2,800 to 18,000. The percentage varies more or less depending upon nature of the enzyme and the hydrolysis conditions.

Though most of the hydrolyzates of the present invention have not so completely been hydrolyzed to amino acids or low molecular peptides, antigenicity of the hydrolyzates is very low because they are almost free of natural conalbumin, ovalbumin, ovomucoid and lysozyme.

(2)   Amino acid composition

Starting albumen and the albumen hydrolyzate according to the invention were respectively treated under a reduced pressure at 110°C for 24 hours in the presence of 6N HCl to accomplish complete hydrolysis to amino acids. Results of measurement of the amino acids are shown in Table 7 in which each value is expressed in ratio on the basis of isoleucine taken as 1.00.

## Table 7

| | Albumen | Albumen hydrolyzate |
|---|---|---|
| Aspartic acid | 1.73 | 1.91 |
| Threonine | 0.88 | 0.91 |
| Serine | 1.51 | 1.55 |
| Glutamic acid | 2.08 | 2.18 |
| Glycine | 1.05 | 1.14 |
| Alanine | 1.50 | 1.57 |
| Cystine | 0.24 | 0.25 |
| Valine | 1.25 | 1.32 |
| Methionine | 0.58 | 0.52 |
| Isoleucine | 1.00 | 1.00 |
| Leucine | 1.53 | 1.56 |
| Tyrocine | 0.48 | 0.50 |
| Phenylalanine | 0.81 | 0.72 |
| Lysine | 0.96 | 1.09 |
| Histidine | 0.31 | 0.29 |
| Arginine | 0.65 | 0.76 |
| Proline | 0.74 | 0.77 |

As shown in Table 7, amino acid composition of the albumen hydrolyzates of the present invention is similar to the amino acid composition of the starting albumen. Therefore, the albumen hydrolyzates of the invention can be used as amino acid source equivalent to the starting albumen.

(3) Antigenicity

Antigenicity of the albumen hydrolyzates of the present invention was determined by the ELISA method in comparison with other denatured albumen products.

The ELISA (enzyme-linked immunosorbent assay) as referred to herein is a method of the immunoassay methods carried out with enzyme-labelled antigen or antibody which requires separation of the labelled antibody bound to antigen from the free one (B/F separation) and in which an immunosorbent is employed. The double antibody method is used for the B/F separation.

Antigen level was determined by the ELISA method (wavelength for the detection 492 nm) in which anti-albumen rabbit antiserum was used as the primary antibody and anti-rabbit IgG-peroxidase-bound goat IgG as the secondary antibody. Data for the measured absorbancy, namely antigenicity as calculated in terms of the absorbancy for a dried product of raw albumen taken as 100 are shown in Table 8.

<u>Table 8</u>

|  | Antigenicity |
| --- | --- |
| Dried product of raw albumen | 100 |
| Commercially available dried albumen | 100 |
| Acidic heat-denatured albumen[1] | 89 |
| Ethanolic heat-denatured albumen[2] | 48 |
| Albumen hydrolyzate[3] | 9 |

1) Dried albumen produced according to the method of

Japanese Patent Application Laid-Open-to-Public No. 15644/1986.

2) The soluble portion of dried albumen obtained by heating in 10% ethanol at 75°C for 1 hour.

3) Albumen hydrolyzate of the invention.

As clearly seen from Table 8, antigenicity of the albumen hydrolyzate of the invention is markedly reduced compared with those denatured by the conventional physiochemical means.

Test Example 2

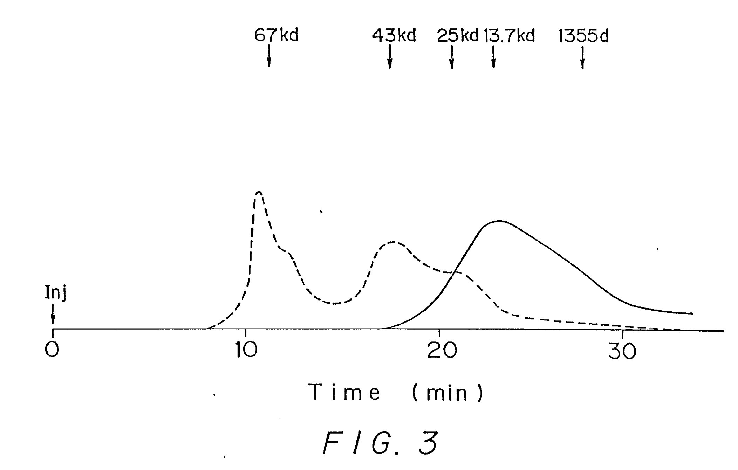

Evaluation of the powdery skim milk hydrolyzate produced by the procedures in Example 2 according to the above-described evaluation method for albumen hydrolyzate was shown in Table 2 above. The recovery ratio was measured using NC Analyzer (manufactured by Sumitomo Kagaku K.K.) for the amount of nitrogen and expressed in percent taking the amount of nitrogen before the reaction as 100. The taste was also tested by 4-grade evaluation (slightly sweet, tasteless, slightly bitter and bitter) in 10 volunteers and described as corresponding to the valuation made by the greatest number of the volunteers. The antigenicity was tested by the ELISA which was carried out using anti-cow milk rabbit serum as the primary antibody and peroxidase-bound anti-rabbit IgG goat serum as the secondary antibody and expressed in relative value taking the absorbancy at 492 nm of powdery skim milk as 100. Furthermore, molecular weight distribution of the hydrolyzate from the one-hour hydrolysis was determined by gel chromatography using HPLC and was shown in Fig. 3. In the figure ⟶ indicates the molecular-weight markers, which were in the order of decreasing molecular weight bovine serum albumin, ovalumin, chymotrypsinogen A, ribonuclease A and cyanocobalamin.

Measurement conditions are as follows (the same also in Test Examples 3-6):

Column: Shodex WS-803

Mobile phase: 0.01 M phosphate buffer solution, 0.15 M NaCl, pH 7.2

Flow rate: 1 ml/min.

Detection: UV 220 nm

The results indicate that the present hydrolyzate represented a molecular population having a molecular weight of 300-30,000, antigenicity of which was reduced to 1/2 that of the original powdery skim milk and which was entirely free of bitter taste.

Test Example 3

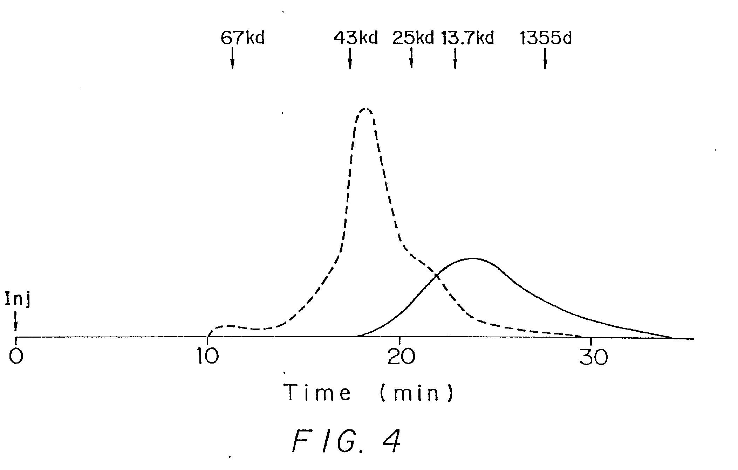

The whey hydrolyzates produced by the procedures in Example 3 were tested by the methods described in Test Example 2. Results are shown in Table 3 above and Fig. 4. Anti-whey rabbit serum was used as the primary antibody (1/800 dilution) and the antigenicity was expressed in value taking the absorbancy at 492 nm for whey as 100.

The figure and the table indicate that the present hydrolyzates represented a molecular population having a molecular weight of 300-34,000 and that the antigenicity was reduced as the hydrolysis time was longer, being about 1/5 that of the original powdery whey after 6 hours. The hydrolyzates were also entirely tasteless.

Test Example 4

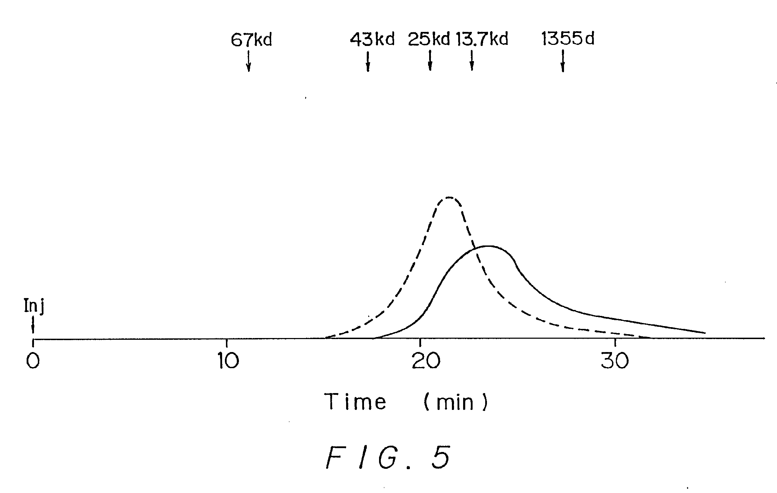

The sardine hydrolyzate produced by the procedures in Example 4 were tested by the methods described in Test Example 2. Results are shown in Table 4 above and Fig. 5. Anti-sardine rabbit serum was used as the primary antibody (1/800 dilution) and the antigenicity was expressed in value taking the absorbancy for dried sample of aqueous sardine extract as 100.

The figure and the table indicate that the present hydrolyzates represented a molecular population having a molecular weight of 400-28,000 and that the antigenicity was decreased as the hydrolysis time was longer and was about 1/50 that of the dried product of aqueous sardine extract after 1 hour.

Test Example 5

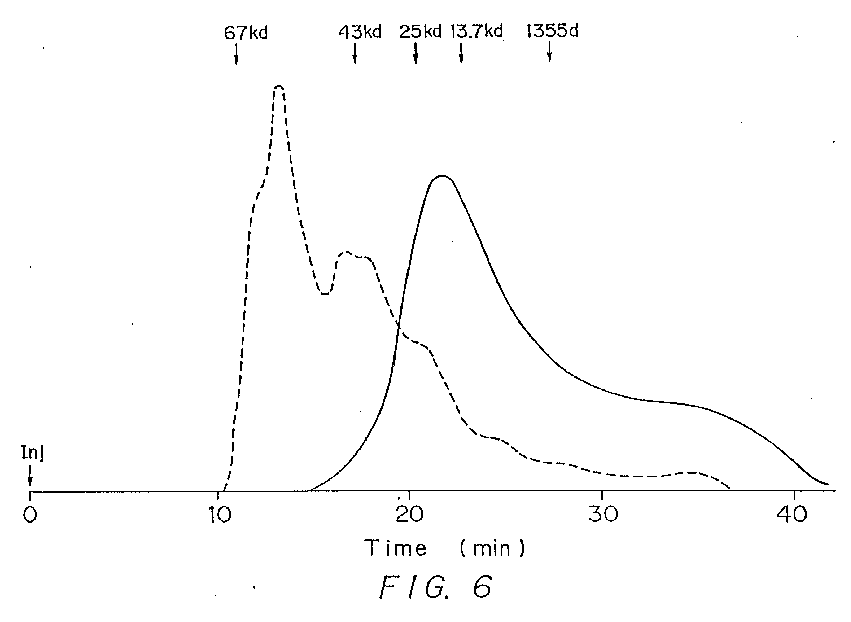

The soybean hydrolyzate produced by the procedures in Example 5 were tested by the methods described in Test Example 2. Results are shown in Table 5 above and Fig. 6. Anti-soybean rabbit serum was used as the primary antibody (1/800 dilution) and the antigenicity was expressed in value taking the absorbancy for dried product of aqueous soybean extract as 100.

The figure and the table indicate that the present hydrolyzate represented a molecular population having a molecular weight of about 50,000 or below with a peak molecular weight of about 18,000 and that though the antigenicity was decreased as the hydrolysis time was longer, they apparently taste bitter with the hydrolyses conducted for 2 hours or longer. On the other hand, the hydrolyzate produced by hydrolysis conducted for 1 hour according to the present method was tasteless and the antigenicity was about 1/20 that of soybean so that it was good as a material for alllergy-care food.

Test Example 6

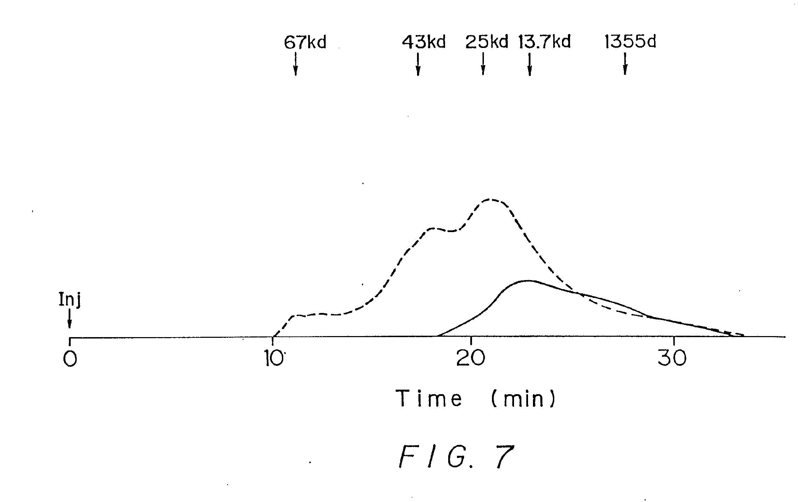

The rice hydrolyzates produced by the procedures in Example 6 were tested by the methods described in Test Example 2. Results are shown in Table 6 above and Fig. 7. Anti-rice rabbit serum was used as the primary antibody (1/800 dilution) and the antigenicity was expressed in value taking the absorbancy for dried product of aqueous rice extract as 100.

The figure and the table indicate that the present hydrolyzate represented a molecular population having a molecular weight of about 28,000 or below with a peak molecular weight of about 17,000 and that the antigenicity was decreased rapidly at one hour after initiation of the hydrolysis and almost completely disappeared at and after 4 hours after the initiation. The taste was slightly sweet. It is believed on the basis of these findings that the present method is favorable as a method for reducing antigenicity of rice protein.

[Referential Example 1]

Products prepared by changing the ethanol concentration in Example 1 were compared with the product of the present invention.

50 g of dried albumen was suspended in 1 lit. of 0-80% ethanol and was heated at 75°C for 1 hour with stirring. Gelatinous albumen thus denatured was subjected to centrifugation followed by freeze-drying. The freeze-dried product was finely divided by a mixer and then re-suspended in 1 lit. of water. To the suspension was added 1 g of Thermoase PC10F, and the mixture was subjected to enzymatic hydrolysis conducted at 70°C for 6 hours with stirring. The aqueous hydrolyzate thus obtained was centrifuged at 3,000 rpm for 10 min. A predetermined amount of protein in the supernatant was developed with gradient gel (10-20%) of SDS polyacrylamide. After silver staining of the gel, a band corresponding to ovalbumin of a molecular weight of about 45,000 was measured by a densitometer (550 nm). Results as expressed taking the value for the albumen without denaturing as 100 were shown in Table 9.

Table 9

Effects of the ethanolic heat denaturation
in enzyme hydrolysis of ovalbumin

| Ethanol concentration (%) | Remaining amount of ovalbumin |
|---|---|
| 0 | 65 |
| 2.5 | 63 |
| 5 | 4 |
| 10 | 0.5 |
| 20 | 0 |
| 40 | 0 |
| 80 | 0 |

As shown in Table 9, it was found that hydrolysis of ovalbumin with Thermoase PC10F was promoted by applying the heat denaturation, more effectively in an ethanol concentration of 5% or higher. The action of ethanol in this treatment is considered to cause such denaturation as being unexpected by heat denaturation alone, for example, due to the change of polarity outward orientation of hydrophobic amino acids which are usually oriented inwardly.

(Referential Example 2)

In order to evaluate antigenicity of the albumen hydrolyzates obtained according to the invention (Example 1) comparison was made with a freeze-dried product of acidic heat-denatured albumen, ethanolic heat-denatured albumen or raw albumen.

The acidic heat-denatured albumen was prepared according to the method described in Japanese Patent Application Laid-Open-to-Public No. 15644/1986 as follows: Albumen from 8 fresh raw eggs is mixed while avoiding bubbling followed by adjustment of pH to 3.5 with 0.5% HCl. The resulting mixture is heated at 75°C for 3 min. and then centrifuged (2,000 rpm) to give gel-like precipitates. To the gel is added an equal amount of water followed by adjustment of pH to 8 with 1% NaOH. The precipitate is solubilized with stirring, and the soluble portion is centrifuged and then freeze-dried to give a freeze-dried product of acidic heat-denatured albumen.

For ethanolic heat-denatured albumen, commercially available dried albumen is dispersed in 10% aqueous ethanol, and the suspension is heated at 75°C for 1 hour and centrifuged. Freeze-drying of the supernatant yields a solid.

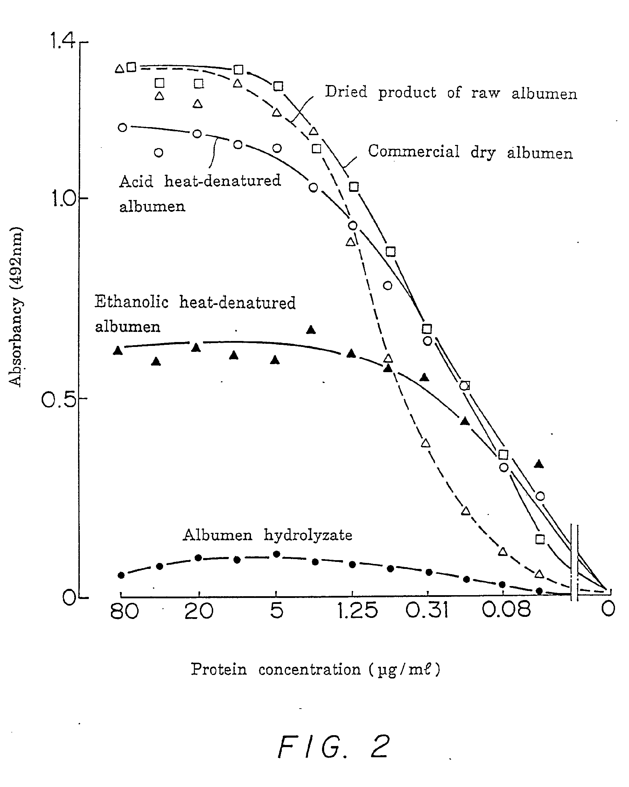

The above-prepared 5 samples were respectively dissolved in 0.05 M carbonate buffer solution (pH 9.6) and analyzed for antigenicity by the ELISA method. On a 96-hole microplate was poured 100 µl of each of the specimens in various protein concentrations to adsorb the protein. Antigen-antibody reaction was conducted using anti-albumen rabbit antiserum (1/1,600 dilution) and anti-rabbit IgG peroxidase-bound goat IgG (1/1,000 dilution). Subsequently, o-phenylenediamine was added, and the color development was expressed in terms of the absorbancy at a wavelength of 492 nm. The larger absorbancy indicates the higher antigenicity. The results are shown in Fig. 2. As shown in Fig. 2, the albumen hydrolyzates of the present invention exhibited very low antigenicity as compared with the other denatured albumen.

Preparative Example using lowly antigenic protein hydrolyzates, particularly lowly antigenic albumen hydrolyzate as protein source

1. Nutritionally balanced beverage (powdery type)

The beverage is prepared by dissolving in 100 ml of distilled water 12-18 g of the powders obtained by procedures as described below. The powders contain per 100 g 2.5 g of soybean oil and 2.5 g of corn oil as fat ingredient (nature of the oil may be changed for soybean allergy or corn allergy), 70-76 g of dextrin as saccharide ingredient and 15 g of the antigenicity-reduced albumen hydrolyzates as protein source. In addition to the above, there is contained a vitamin-mineral mix so as to give contents as specified below.

| | |
|---|---|
| Sodium | (270-350 mg), |
| Potassium | (270-330 mg), |
| Calcium | (270-330 mg), |
| Chlorine | (540-660 mg), |
| Phosphorus | (160-250 mg), |
| Magnesium | ( 77- 95 mg), |
| Copper | (0.17-0.23 mg), |
| Manganese sulfate | (2.3-2.9 mg), |
| Zinc sulfate | (5.3-7.9 mg), |

| | |
|---|---|
| Iron gluconate | ( 21- 29 mg), |
| Retinol palmitate | (900-1300 IU), |
| Thiamin chloride | (0.90-1.15 mg), |
| Riboflavin | (0.90-1.15 mg), |
| Pyridoxin hydrochloride | (0.90-1.15 mg), |
| Cyanocobalamin | (1.5-2.5 µg), |
| Ascorbic acid | (180-240 mg), |
| Cholecalciferol | ( 80-120 IU), |
| Tocopherol acetate | ( 27- 34 IU), |
| Nicotinamide | (9.0-11.5 mg), |
| Calcium pantothenate | (1.8-2.3 mg), |
| Folic acid | (0.18-0.23 mg), |
| Biotin | ( 45- 58 µg), |
| Choline chloride | ( 18- 23 mg), |
| Inositol | ( 18- 23 mg) |

The powders were prepared as follows:  To fat-soluble soybean oil, corn oil, retinol palmitate, cholecalciferol and tocopherol acetate are added glycerin, glycerin fatty acid esters and hydroxypropylcellulose.  The mixture with distilled water added is homogenized in a homogenizer.  To the liquid mixture is added a solution of dextrin in distilled water, and the resulting mixture is well blended and spray-dried.  With the dry product thus produced are mixed the antigenicity-reduced albumen hydrolyzate, sodium chloride, potassium chloride, sodium citrate, calcium glycerophosphate and iron gluconate so as to produce the above-mentioned composition.  Onto the mixture fluidized by the fluidized drying granulation method under sterilized warm air are successively sprayed an aqueous solution of magnesium chloride, magnesium sulfate, copper sulfate, manganese sulfate and zinc sulfate and an aqueous solution of thiamin hydrochloride, riboflavin, pyridoxin hydrochloride, cyanocobalamin, ascorbic acid, nicotinamide, calcium pantothenate, folic acid, biotin, choline chloride and inositol. Granulation and vacuum dehydration are followed.

## 2. Mayonnaise-like dressing

To 10 ml of distilled water are added 8 g of the antigenicity-reduced albumen hydrolyzate, 3 g of powdery mustard, 0.5 g of pepper, 1.5 g of sodium chloride and 0.5 g of yolk lecithin PL-100 (manufactured by Kewpie K.K.). The mixture is well bubbled in a bubbler followed by addition of 6 ml of apple vinegar and then mixed. To the resulting liquid mixture are portionwise added 40 g of safflower oil and 30 g of olive oil using a powerful mixer. There was obtained a mayonnaise-like dressing.

## 3. Pumpkin cookie

To a mixture of 20 g of the antigenicity-reduced albumen hydrolyzate, 30 g of powdery oil (corn 1, safflower 1), 15 g of distilled water, 25 g of sugar, 0.5 g of sodium chloride, 10 g of liquid sugar and 10 g of pumpkin paste prepared while bubbling are added 80 g of barley flour, 20 g of wheat flour (potato starch or the like may be used in the case of wheat allergy) and 1.5 g of baking powders that have been screened, and the resulting mixture is roughly blended. The blend is baked in an oven at 170-180°C to scorching.

4. Retort-pouched pudding

In 75 g of water are stirred 2 g of the antigenicity-reduced albumen hydrolyzate, 5 g of powdery skim milk (this can not be used in the case of milk allergy), 12 g of sugar, 4 g of purified coconut oil, 1 g of corn starch, 0.5 g of a gelling agent and 0.1 g of an emulsifier at 80°C for 10 min. to a solution. Then, 0.1 g of carotin and 0.3 g of a flavoring are added. The resulting mixture is homogenized in a high-pressure homogenizer (150 kg/cm$^2$). The homogenate is filled in containers, retort sterilized at 120°C for 20 min. and solidified by cooling.

5. Coffee jelly

With 70 g of distilled water are mixed 10 g of sugar, 10 g of glucose, 2 g of the antigenicity-reduced albumen hydrolyzate and 1 g of a gelling agent with stirring. The mixture is stirred at 80°C for 10 min. to a solution. To the solution are added 8 g of a coffee extract and 0.2 g of a flavoring. The resulting mixture is filled in containers and solidified by cooling.

6. Tableted candy

A mixture of 85.5 g of powdery sugar, 5 g of corn starch, 5 g of L-tartaric acid, 4 g of a thickener (0.5% Vistop D-20, manufactured by San-ei Kagaku K.K.) and 0.5 g of DK ester is granulated through a screen, $\phi$1.0-$\phi$1.5 mm in mesh. The granules are dried at 60°C for 30-40 min. to a water content of 1-0.5%. With 97 g of the granules are blended 0.5 g of talc, 0.5 g of DK ester, 0.5 g of lemon extract and 2.5 g of the antigenicity-reduced albumen hydrolyzate, and the blend is tableted.

7.  Lemon cake

To 20 g of the antigenicity-reduced albumen hydrolyzate are added 150 g of sugar and 70 ml of water. The mixture is bubbled by a bubbler in a steam bath at 50°C. To the mixture is added 20 g of a thickner (Vistap D-907 manufactured by San-ei Kagaku K.K. or the like) followed by additional bubbling to such a thickening that drops from the bubbler draw double circules on the surface.  To the bubbled mass were added juice and grated pericarp of half a lemon and then 120 g of barley flour and 30 g of solft flour through a screen. When smoothly fluidized, a melt of 100 g of palm kernel oil and 30 g of safflower oil is poured, and the resulting mass is blended and baked at about 165°C for 25-30 min.

As described above, lowly antigenic foods can be prepared by using as protein source a hydrolyzate produced by the method for preparing protein hydrolyzate of the present invention.  In addition to the above, a variety of lowly antigenic foods can also be prepared using the hyrolyzate as protein source for foods such as ice cream, sherbet, "furikake" (chipped seasoning for plain cooked rice), steamed egg custard, pot-steamed hotchpotch, cream, marshmallow and cake.

Industrial Applicability

According to the present invention there can be obtained novel protein hydrolyzates of very low antigenicity by destroying antigenic determinants of natural antigenicity-holding proteins such as conalbumin, ovalbumin, ovomucoid and lysozyme without undue decomposition of the proteins to amino acids and low-molecular peptides.

The albumen hydrolyzates of the present invention are also highly digestive and absorptive because they are almost composed of oligopeptides and low-molecular peptides. As the amino acid composition is also the same as that of the starting protein and usable as amino acid source of value equal to that of the starting protein.

Therefore, they can safely be given as protein source to food allergic patients, sucklings and little children as well as pregnant women and nursing mothers who are known to influence upon the fetus and suckling via their blood and milk. As food allergy is frequent in sucklings and little children who are premature in digestive or adsorptive ability, the protein hydrolyzates of the present invention are useful for them.

The use as proteinous material is also expected for elder persons suffering from reduced immunological, digestive or absorptive ability and patients temporarily suffering from such reduced ability.

As a matter of fact, the protein hydrolyzates of the present invention are proteinous materials for food allergic patients retaining the high nutritional value of the starting proteins but with reduced antigenicity. As described above, they are useful in the fields of food and medical industries.

Brief Description of the Drawings

Fig. 1 is a graphic demonstration of molecular weight distribution of albumen hydrolyzates of the present invention by gel chromatography.

Fig. 2 is a graphic demonstration of antigenicity of various samples by the ELISA method. In Fig. 2,

● -●: The albumen hydrolyzate

▲ -▲: Ethanolic heat-denatured albumen

○ -○: Acidic heat-denatured albumen

△ -△: Dried product of raw albumen

□ -□: Commercially available dry albumen

respectively indicate antigenicity.

Fig. 3 is a graphic demonstration of molecular weight distribution of powdery skim milk hydrolyzates by gel chromatography, Fig. 4 of whey hydrolyzates, Fig. 5 of sardin hydrolyzates, Fig. 6 of soybean hydrolyzates and Fig. 7 of rice hydrolyzates. In Figs. 3-7, dotted line and solid line indicate molecular weight distribution of the protein in the starting material and the hydrolyzate (one-hour hydrolysis), respectively.

## Claim

1)      An albumen hydrolyzate substantially free of ovalbumin and ovomucoid and containing 95% by weight or more of a protein hydrolyzate of a molecular weight distribution in a range of 2,800 to 18,000 as determined by gel chromatography.

2)      An albumen hydrolyzate with antigenicity reduced to a half or below of that of the albumen per unit protein as determined by ELISA (enzyme linked immunosorbent assay) method using anti-albumen serum.

3)      An antigenicity-reduced food comprising an albumen hydrolyzate according to Claim 1 or 2.

4)      A method for preparing protein hydrolyzates which comprises denaturing a proteinous substance by heating in a polar hydrophilic organic solvent followed by hydrolysis with a bacterial protease.

5)      A method for preparing protein hydrolyzate according to Claim 4 wherein the heating temperature is 50-95°C.

6)      A method for preparing protein hydrolyzate according to Claim 4 wherein the proteinous substance is albumen protein.

7)      A method for preparing protein hydrolyzates according to Claim 4 wherein the proteinous substance is dairy protein.

8)      A method for preparing protein hydrolyzates

EP 0 357 776 A1

according to Claim 4 wherein the proteinous substance is cereal protein.

9) A method for preparing protein hydrolyzates according to Claim 4 wherein the proteinous substance is meat protein.

10) A preparative method according to Claim 4 wherein the polar hydrophilic organic solvent is a lower alcohol.

11) A preparative method according to Claim 4 wherein the bacterial protease is a heat-resistant neutral protease.

12) A method for preparing protein hydrolyzates which comprising denaturing a proteinous substance by heating in a polar hydrophilic solvent followed by hydrolysis with a bacterial protease, subjecting the hydrolyzate to centrifugation and collecting the supernatant.

13) A method for preparing protein hydrolyzates according to Claim 12 wherein the heating temperature is 50-95°C.

Eluted amount (mℓ)

FIG. 1

FIG. 2

67kd      43kd   25kd 13.7kd    1355d

Inj

Time (min)

FIG. 3

EP 0 357 776 A1

67kd  43kd 25kd 13.7kd 1355d

Inj

0   10   20   30

Time (min)

*F I G. 4*

EP 0 357 776 A1

FIG. 5

FIG. 6

EP 0 357 776 A1

67kd     43kd   25kd 13.7kd    1355d

Inj

0          10          20          30

Time (min)

FIG. 7

EP 0 357 776 A1

# INTERNATIONAL SEARCH REPORT

International Application No PCT/JP88/00460

## I. CLASSIFICATION OF SUBJECT MATTER (if several classification symbols apply, indicate all) [6]

According to International Patent Classification (IPC) or to both National Classification and IPC

Int.Cl[4] C07K15/00, C12P21/06, A23J3/00//A23L1/06,
1/24, 1/187, 2/38, A21D13/08, A23G3/00

## II. FIELDS SEARCHED

| Minimum Documentation Searched [7] | |
|---|---|
| Classification System | Classification Symbols |
| IPC | C07K15/00, C12P21/06, A23J3/00 |

| Documentation Searched other than Minimum Documentation to the Extent that such Documents are Included in the Fields Searched [8] |
|---|
| |

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| X | Agric, Biol. Chem. Vol.49, No.7 (1985) [Reduction of Ovomucoid Immunogenic Activity on Peptic Fragmentation and Heat Denaturation] P2237-2241 | 1-3 |
| X | JP, B1, 47-41019 (Kikkoman Corporation) 17 October 1972 (17. 10. 72) (Family: none) | 4-13 |
| X | JP, B1, 47-22795 (Kikkoman Corporation) 26 June 1972 (26. 06. 72) (Family: none) | 4-13 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 2, 1988 (02. 08. 88) | August 15, 1988 (15. 08. 88) |
| International Searching Authority | Signature of Authorized Officer |
| Japanese Patent Office | |

Form PCT/ISA/210 (second sheet) (January 1985)